(19) Europäisches Patentamt
European Patent Office
Office européen des brevets

(11) EP 1 415 643 A1

(12) # DEMANDE DE BREVET EUROPEEN

(43) Date de publication:
**06.05.2004 Bulletin 2004/19**

(51) Int Cl.[7]: **A61K 7/13**

(21) Numéro de dépôt: **03292596.8**

(22) Date de dépôt: **17.10.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **21.10.2002 FR 0213097**

(71) Demandeur: **L'OREAL**
**75008 Paris (FR)**

(72) Inventeurs:
- **Plos, Grégory**
  **75011 Paris (FR)**
- **Guerin, Fréderic**
  **75009 Paris (FR)**

(74) Mandataire: **Casalonga, Axel**
**BUREAU D.A. CASALONGA - JOSSE**
**Paul-Heyse-Strasse 33**
**80336 München (DE)**

(54) **Procédé de coloration rapide et de décoloration rapide de fibres kératiniques humaines avec des colorants directs**

(57) L'invention concerne un procédé de coloration rapide pour fibres kératiniques humaines, et en particulier les cheveux, consistant à appliquer sur ces fibres au moins une composition tinctoriale contenant dans un milieu approprié pour la teinture au moins un colorant direct choisi parmi les colorants aryles méthaniques, les azoïques cationiques, les méthiniques et azométhiniques, ainsi que les azines ; à maintenir la composition au contact des fibres pendant un temps de pose inférieur à 5 minutes ; puis à rincer les fibres traitées ; la coloration présentant selon la notation CIELAB une valeur de L* inférieure à 40 et/ou une valeur de C* supérieure à 20 et de préférence supérieure à 25, lorsque la composition est appliquée sur les cheveux à 90% blancs naturels à la température de 27°C ± 5°C pendant une durée de 4 minutes pour un rapport de bain de 10.

L'invention concerne également un procédé de décapage de la fibre colorée par les colorants directs précédemment cités, par application d'un traitement oxydant ou réducteur en un temps inférieur à 5 minutes.

EP 1 415 643 A1

Printed by Jouve, 75001 PARIS (FR)

## Description

La présente invention a pour objet un procédé de coloration rapide des fibres kératiniques humaines en particulier des cheveux avec certains colorants directs ainsi que leur décoloration avec certains colorants directs.

Dans le cadre de la vie moderne et active, de nombreuses personnes souhaitent changer de couleur de cheveux, mais ne s'y tiennent pas en raison d'un manque de temps. Les produits de coloration actuellement disponibles sur le marché ne permettent pas de teindre les cheveux de façon significative dans des temps de pose très courts, puisqu'ils nécessitent généralement pour le moins entre 20 et 30 minutes pour atteindre un résultat tinctorial appréciable.

Certaines personnes souhaitent également changer très fréquemment de couleur du cheveu, ce qui est difficilement réalisable pour des raisons techniques, puisqu'il faut qu'elles passent par une étape de décapage en condition oxydante ou réductrice généralement lente.

La demanderesse vient de découvrir qu'il était possible d'obtenir des colorations intenses de façon très rapide dans des délais inférieurs à 5 minutes même sur cheveux non sensibilisés en mettant en oeuvre certains colorants directs et plus particulièrement choisis parmi des colorants aryle méthaniques, azoïques cationiques, des méthiniques et azométhiniques, ainsi que aziniques.

La demanderesse a pu constater que l'on obtient ainsi des colorations intenses très rapidement, même sur des cheveux non sensibilisés. Elle a par ailleurs constaté que ces colorants pouvaient être éliminés très rapidement et les cheveux pouvaient retrouver leur coloration d'origine, sans que cela ne gène les colorations ultérieures, par application d'un traitement oxydant ou réducteur, et ceci également dans des temps très courts, inférieurs à 5 minutes.

L'invention a donc pour objet un procédé de coloration des fibres kératiniques humaines en particulier des cheveux mettant en oeuvre ces colorants directs en ayant des temps de pose très courts, inférieurs à 5 minutes.

D'autres objets de l'invention apparaîtront à la lecture de la description et des exemples qui suivent.

Le procédé conforme à l'invention consiste à appliquer, sur des fibres kératiniques humaines et plus particulièrement les cheveux au moins une composition tinctoriale contenant dans un milieu approprié pour la teinture au moins un colorant direct choisi parmi les colorants aryle méthaniques, azoïques cationiques, des méthiniques et azométhiniques, ainsi que aziniques ; à maintenir la composition au contact des fibres pendant un temps de pose inférieur à 5 minutes ; puis à rincer la fibre traitée ; la coloration présentant selon la notation CIELAB une valeur de L* inférieure à 40 et/ou une valeur de C* supérieure à 20, lorsque la composition est appliquée sur les cheveux à 90% blancs naturels à la température de 27°C ± 5°C pendant une durée de 4 minutes pour un rapport de bain de 10.

De préférence, les colorants sont choisis parmi ceux conduisant à 0,5% en poids à une valeur de L* inférieure à 40 et/ou une valeur de C* supérieure à 25 pour un temps de pose de 1 minute sur cheveux à 90% blancs naturels, à la température ambiante (27°C ± 5°C) pour un rapport de bain de 10.

La notation CIELAB définit un espace colorimétrique dans lequel chaque couleur est définie par 3 paramètres (L*, a*, b*) :

- le paramètre L* reflète la clarté de la couleur , la valeur L* étant égale à 0 pour le noir et égale à 100 pour le blanc absolu. Plus la valeur de L* est élevée, moins la coloration est intense,
- le paramètre a* correspond à l'axe du couple antagoniste vert/rouge
- le paramètre b* correspond à l'axe du couple antagoniste bleu/jaune
- la valeur du paramètre C* ou index de chromaticité est égale à $\sqrt{(a^{*2}+b^{*2})}$

Les colorants aryle méthaniques sont plus particulièrement choisis parmi les composés répondant aux structures suivantes (I), (II), (III) et (IV) :

- les composés de triaminotriphénylméthane

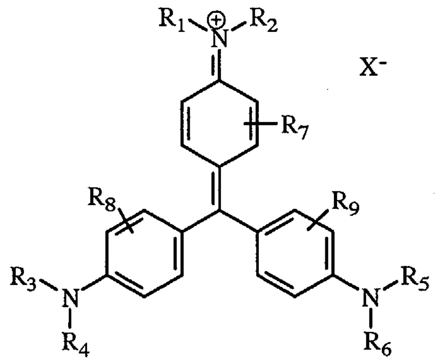

(I)

dans laquelle $R_1$, $R_2$, $R_3$, $R_4$, $R_5$ et $R_6$ indépendamment les uns des autres peuvent être un atome d'hydrogène, un groupement alkyle en $C_1$-$C_4$ tel que les groupements méthyle ou éthyle ou un groupement mono ou poly hydroxyalkyle en $C_1$-$C_4$ tel que le groupement β-hydroxyéthyle, un groupement phényle, ou un groupement benzyle,

$R_7$, $R_8$, $R_9$ indépendamment les uns des autres peuvent être un atome d'hydrogène, un atome d'halogène tel le chlore ou un groupement alkyle en $C_1$-$C_4$ tel que méthyle ou éthyle.

A titre d'exemple, on peut citer le Basic Red 9 (Basic Fuchsin C. I. 42500), le Basic Violet 1 (Méthyl Violet 2B C.I.42535), le Basic Violet 2 (New Fuchsin C.I. 42520), l'Opal Blue SS.

- les composés de diaminotriphénylméthane

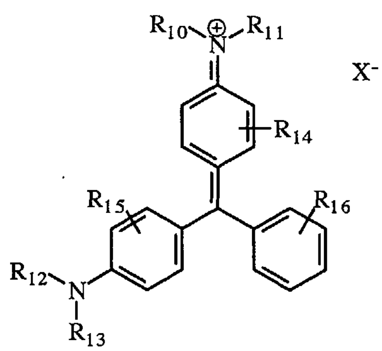

(II)

dans laquelle $R_{10}$, $R_{11}$, $R_{12}$, et $R_{13}$ indépendamment les uns des autres, peuvent être un atome d'hydrogène, un groupement alkyle en $C_1$-$C_4$ tel que les groupements méthyle, éthyle ou un groupement mono ou poly hydroxyalkyle en $C_1$-$C_4$ tel que le groupement β-hydroxyéthyle,

$R_{14}$ et $R_{15}$, indépendamment l'un de l'autre, peuvent être un atome d'hydrogène ou un groupement alkyle en $C_1$-$C_4$ tel que méthyle ou éthyle, ou un groupement mono ou poly hydroxyalkyle en $C_1$-$C_4$ tel que le groupement β-hydroxyéthyle,

$R_{16}$ peut être un atome d'hydrogène, un atome d'halogène tel que le chlore, un groupement alkyle en $C_1$-$C_4$ tel que méthyle ou éthyle, ou un groupement mono ou polyhydroxyalkyle en $C_1$-$C_4$ tel que β-hydroxyéthyle.

A titre d'exemple, on peut citer le Basic Blue 1 (C.I.42025), le Basic Green 1 (Brilliant Green 1 C.I.42040), le Basic Green 4 (Malachite Green Oxalate C.I.42000), le Basic Blue 5 (Brillant Glacier Blue C.I.42140).

- les composés de triaminonaphtyldiphénylméthane

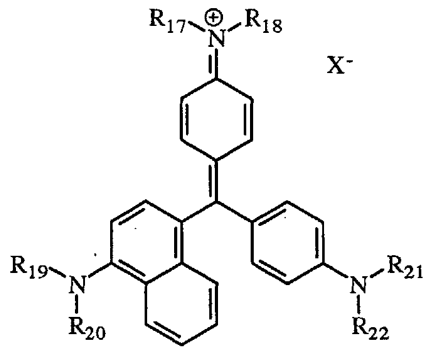

(III)

dans laquelle $R_{17}$, $R_{18}$, $R_{21}$, et $R_{22}$ indépendamment les uns des autres, peuvent être un groupement alkyle en $C_1$-$C_4$ tel que les groupements méthyle, éthyle ou un groupement mono ou poly hydroxyalkyle en $C_1$-$C_4$ tel que le groupement β-hydroxyéthyle,

$R_{19}$ et $R_{20}$, indépendamment l'un de l'autre, peuvent être un atome d'hydrogène, un groupement alkyle en $C_1$-$C_4$ tel que le groupement éthyle, un groupement mono ou poly hydroxyalkyle en $C_1$-$C_4$ tel que le groupement β-hydroxyéthyle, un groupement phényle, un groupement benzyle ou un groupement toluyle.

A titre d'exemple, on peut citer le Basic Blue 11 (Victoria Blue R C.I.44040), le Basic Blue 15 (Bleu de nuit C. I.44085), le Basic Blue 26 (Victoria blue B C.I.44045).

- des composés de monoaminotriphénylméthane

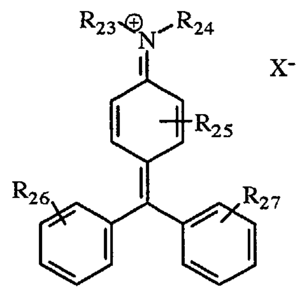

(IV)

dans laquelle $R_{23}$, $R_{24}$, indépendamment l'un de l'autre, peuvent être un atome d'hydrogène, un groupement alkyle en $C_1$-$C_4$ tel que les groupements méthyle ou éthyle, un groupement mono ou poly hydroxyalkyle en $C_1$-$C_4$, ou un groupement benzyle,

$R_{25}$, $R_{26}$ et $R_{27}$ indépendamment les uns des autres, peuvent être un atome d'hydrogène, un atome d'halogène tel que le chlore ou un groupement alkyle en $C_1$-$C_4$ tel que les groupements méthyle ou éthyle.

A titre d'exemple, on peut citer la fuchsonimine hydrochloride (CAS# 84215-84-9).

Les structures des colorants directs azoïques monocationiques et polycationiques utilisables conformément à l'invention sont données ci-dessous :

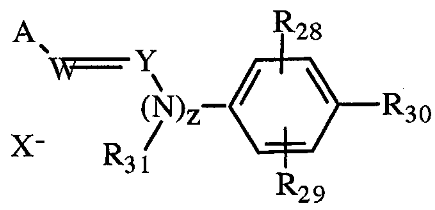

(V)

où A représente l'une des structures suivantes :

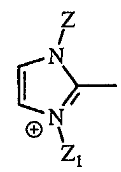 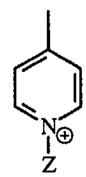 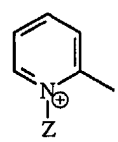 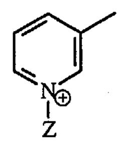 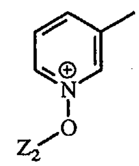

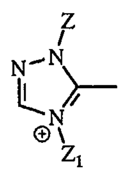 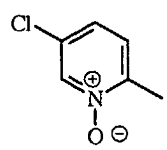

et dans laquelle W et Y représentent indépendamment l'un de l'autre, un atome d'azote ou un atome de carbone,

Z, $Z_1$, $Z_2$ désignent indépendamment les uns des autres un radical alkyle en $C_1$-$C_4$ et en particulier le méthyl,

$R_{28}$ et $R_{29}$ indépendamment l'un de l'autre représentent un atome d'hydrogène, un atome d'halogène, un groupement alkyle linéaire ou ramifié en $C_1$-$C_4$ ou un groupement alkoxy linéaire ou ramifié en $C_1$- $C_4$, ou un groupement phénol permettant de former un enchaînement naphtalène avec le groupe phényle adjacent,

z est un nombre entier qui peut être 0 ou 1,

$R_{30}$ représente un atome d'hydrogène, un groupement alkoxy linaire ou ramifié en $C_1$-$C_4$, un groupement $NR_{32}R_{33}$ dans lequel $R_{32}$ et $R_{33}$ indépendamment l'un de l'autre représentent un atome d'hydrogène ou un groupement alkyle linéaire ou ramifié en $C_1$-$C_4$, un groupement toluyle, un groupement mono ou poly hydroxyalkyle tel que le groupement hydroxyéthyle, un groupement $-CH_2SO_3Na$, un groupement benzyle ou forme avec l'atome d'azote adjacent et un atome de carbone du noyau benzénique un hétérocycle,

$R_{31}$ représente un atome d'hydrogène, un groupement alkyle linéaire ou ramifié en $C_1$-$C_4$, ou forme avec l'atome d'azote adjacent et un atome de carbone du noyau benzènique un hétérocycle.

A titre d'exemple, on peut citer le Basic Red 46 (C.I. 110825)

Les structures des colorants méthiniques et azométhiniques dérivées d'un noyau indolinium ou benzothiazolinium utilisables conformément à l'invention de formules (VI), (VII) et (VIII) sont données ci-dessous :

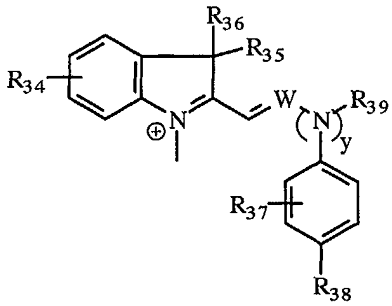

(VI)

dans laquelle $R_{34}$, $R_{35}$ et $R_{36}$, indépendamment les uns des autres, représentent un atome d'hydrogène ou un groupement en $C_1$-$C_4$ tel que le groupement méthyle,

W peut être un atome de carbone ou d'azote,

y représente un nombre entier qui peut être 0 ou 1,

$R_{37}$ peut être un atome d'hydrogène, un groupement alkyle en $C_1$-$C_4$ tel que le méthyle ou un groupement alkoxy en $C_1$-$C_4$ tel que le groupement méthoxy,

$R_{38}$ peut être un atome d'hydrogène, un groupement méthoxy, un groupement $NR_{40}R_{41}$ où $R_{40}$ et $R_{41}$, indépendamment l'un de l'autre, peuvent être un groupement alkyle en $C_1$-$C_4$ tel que méthyle, éthyle ou propyle éventuellement substitué par un atome de chlore ou par un groupement cyano,

$R_{39}$ peut être un atome d'hydrogène, un groupement alkyle en $C_1$-$C_4$ tel que le groupement méthyle, ou forme un hétérocycle avec les atomes de carbone du noyau benzénique adjacent.

A titre d'exemple, on peut citer le Basic Red 13 (C.I. 48015), le Basic Red 14 (C.I. 48016), le Basic Violet 7 (C.I. 48020), le Basic Yellow 23 (C.I. 48100), le Basic Yellow 28 (C.I. 48054).

De même, le colorant méthinique Basic Orange 21 (C.I.48035) de formule (VII) peut être utilisé conformément à l'invention.

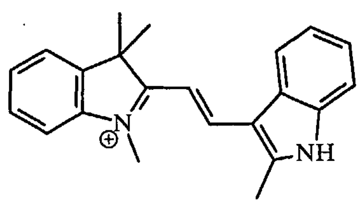

(VII)

Il est également possible d'utiliser conformément à l'invention la carbocyanine de formule (VIII) :

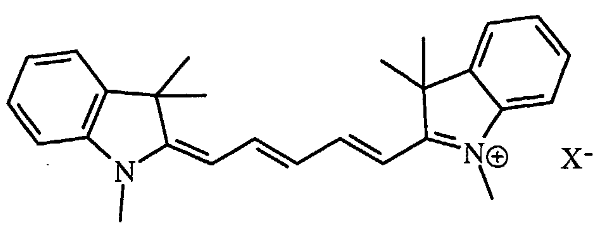

(VIII)

Des dérivés de benzothiazole de formule (IX) peuvent aussi être utilisés pour cette application :

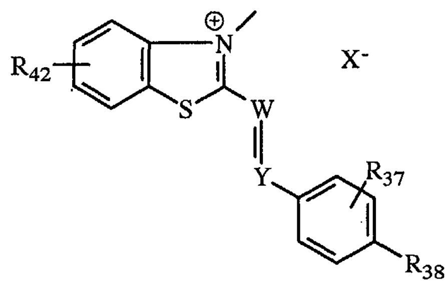

(IX)

dans laquelle $R_{42}$ peut être un atome d'hydrogène, un groupement alkyle en $C_1$-$C_4$ tel que méthyle ou éthyle, un groupement alcoxy en $C_1$-$C_4$ tel que le groupement méthoxy,

$R_{37}$ et $R_{38}$ ont la même signification que dans la structure de formule (VI),

W et Y, indépendamment l'un de l'autre, peut être un atome de carbone ou d'azote.

A titre d'exemple, on peut citer le Basic Blue 41 (C.I. 11105),

Les structures aziniques, comprenant les phénazines (formule X), les azophénazines (formule XI), les thiazines (formule XII) ainsi que les oxazines (formule XIII) utilisables conformément à l'invention sont données ci-dessous :

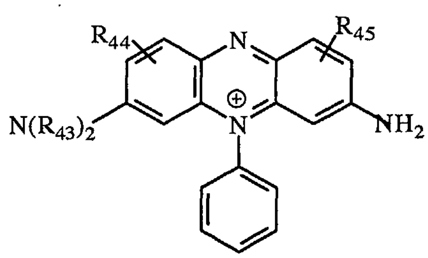

(X)

dans laquelle $R_{43}$, $R_{44}$ et $R_{45}$, indépendamment les uns des autres, représentent un atome d'hydrogène, un groupement alkyle en $C_1$-$C_4$ tel que méthyle ou éthyle.

A titre d'exemple, on peut citer le Méthylène Violet 3RAX (C.I.50206).

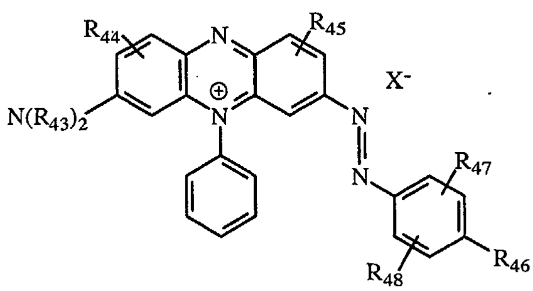

(XI)

dans laquelle $R_{43}$, $R_{44}$ et $R_{45}$ ont la même signification que dans la formule (X)

$R_{46}$ représente un atome d'hydrogène, un groupement hydroxyle ou un groupement amino,

$R_{47}$ et $R_{48}$, indépendamment l'un de l'autre, peuvent être un atome d'hydrogène, un groupement hydroxyle ou un groupement phényle permettant de former un enchaînement naphtalène avec le groupement phényle adjacent.

A titre d'exemple, on peut citer le Basic Blue 16 (C.I.12210), le Basic Black 2 (C.I.11815), le Janus Green B (C.I.11050).

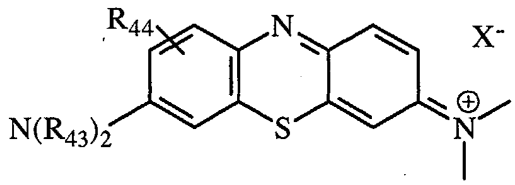

(XII)

dans laquelle $R_{43}$ et $R_{44}$ ont la même signification que dans la formule (X)

A titre d'exemple, on peut citer le Basic Blue 17 (C.I.52040), le Basic Blue 9 (C.I. 52015).

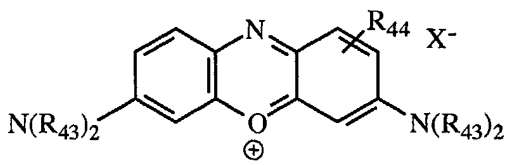

(XIII)

dans laquelle $R_{43}$ et $R_{44}$ ont la même signification que dans la formule (X)

A titre d'exemple, on peut citer le Basic Blue 3 (C.I. 51004).

Dans chacune des structures ci-dessus, $X^-$ représente un anion qui peut être issu d'un atome d'halogène, préférentiellement l'atome chlore, ou les ions $HSO_4^-$, méthosulfate, benzoate ou acétate.

Les colorants Basic Red 22, Basic Blue 7 et Basic Violet 14 ne font pas partie des colorants directs utilisables selon l'invention.

La concentration en colorants directs est généralement comprise entre 0,001% et 10%, et préférentiellement entre 0,05% et 5%. Cette concentration dépend de l'intensité de coloration voulant être obtenue.

La température d'application est comprise généralement entre la température ambiante et 80°C, et préférentiellement entre la température ambiante et 60°C, de préférence les cheveux sont teints à une température de 27°C ± 5°C.

Le temps d'application est généralement compris entre 30 secondes et moins de 5 minutes, et préférentiellement compris entre 1 minute et 3 minutes, et encore plus préférentiellement entre 1 minute et 2 minutes.

Les cheveux sont ensuite rincés à l'eau.

La gamme de pH pour la composition tinctoriale est compris préférentiellement entre 2 et 11, et en particulier entre 3 et 11.

Les compositions sont appliquées sur tout type de cheveux, en particulier sur des cheveux ayant subi un traitement de décoloration. Les compositions utilisées conformément à l'invention peuvent être sous forme de mousse (aérosol), de crème, de gel, de lotion ou encore de shampooing.

On peut utiliser dans les compositions des adjuvants cosmétiques classiques utilisés dans les compositions tinctoriales tels que : les tensio-actifs anioniques, cationiques, non-ioniques et/ou amphotères, les polymères épaississants, les agents conditionneurs (polymères cationiques, cations, silicones, ....), les solvants, les agents alcalins, ainsi que les agents acides.

Le décapage oxydant s'effectue par tout traitement oxydant en particulier par application de peroxyde d'hydrogène ou des composés susceptibles de produire par hydrolyse du peroxyde d'hydrogène, tels que le peroxyde d'urée ou les persels comme les perborates, les percarbonates et les persulfates, le peroxyde d'hydrogène étant particulièrement préféré.

Ce décapage est d'autant plus efficace que le pH de la composition de décapage est alcalin.

On peut également utiliser un décapage en milieu réducteur, de préférence acide. A titre de réducteur utilisable, on peut citer les sulfites, l'hydrosulfite, les sulfinates.

Le décapage se caractérise par sa rapidité, moins de 5 minutes pour les structures envisagées, quelle que soit la nature du cheveu. Le décapage n'entraîne ni dommage, ni éclaircissement de la fibre capillaire, de par le court temps de pose.

Après décapage, les cheveux peuvent être recolorés sans perte du pouvoir tinctorial.

Les exemples suivants sont destinés à illustrer l'invention sans présenter un caractère limitatif.

Exemple 1

Les colorants mentionnés ci-dessus ont été introduits à la concentration de 0,5% dans le support ci-dessous :

| Composé | Concentration |
|---|---|
| Alcool benzylique | 10% |
| Polyéthylène glycol (8 OE) | 12% |
| Hydroxyéthyl cellulose P. M. 720 000 vendu par la société Aqualon | 1,5% |
| Colorant direct * | 0,5% |
| pH | spontané |
| Eau distillée | q.s.p 100% |

Les colorants ont été choisis parmi :

- les composés triarylméthanes : Basic Blue 1, Basic Blue 11, Basic Green 1, Basic Violet 1, Basic Violet 2, Basic Violet 4,
- les composés azoïques cationiques : Basic Red 46

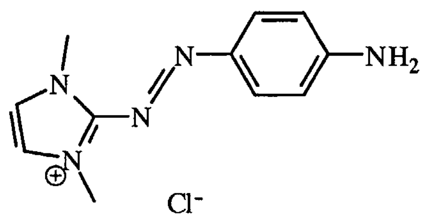

Basic red 51

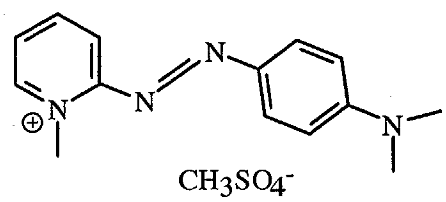

Colorant B

- les composés cyanines méthiniques : Basic Red 14, Basic Yellow 13, Basic Yellow 23, Basic Yellow 28,

Ces compositions ont été appliquées à la température de 27°C ± 5°C sur des mèches de cheveux à 90% blancs naturels de 1 gramme avec un rapport de bain 10 pendant 1 minute. Ensuite, les mèches ont été rincées à l'eau claire, shampouinées et séchées. Après séchage, les mèches ont été lues au spectrocolorimètre CM 2002-Minolta (angle 10%, composantes spéculaires exclues, illuminant D65) dans le système CIEL*a*b*. Les valeurs L*a*b* de la mèche témoin sont : L* = 58,16 ; a* = 0,86 ; b* = 10,5.

Les résultats colorimétriques sont donnés ci-dessous :

| Colorant | L* | a* | b* | C* |
|---|---|---|---|---|
| Basic Blue 1 | 37,02 | -23,22 | -8,75 | 24,81 |
| Basic Blue 11 | 27,97 | 8,74 | -23,64 | 25,20 |
| Basic Green 1 | 38,75 | -29,20 | 0,20 | 29,20 |
| Basic Violet 1 | 27,41 | 15,34 | -19,27 | 24,63 |
| Basic Violet 2 | 31,20 | 23,34 | -3,56 | 23,61 |
| Basic Violet 4 | 27,78 | 13,99 | -27,83 | 31,15 |
| Basic Red 51 | 32,29 | 33,16 | 2,74 | 33,27 |
| Colorant B | 26,22 | 23,72 | -15,87 | 28,54 |
| Basic Red 46 | 36,84 | 34,25 | 3,65 | 34,46 |
| Basic Red 14 | 43,34 | 34,23 | 3,56 | 34,41 |

(suite)

| Colorant | L* | a* | b* | C* |
|---|---|---|---|---|
| Basic Yellow 13 | 55,83 | -4,85 | 41,21 | 41,45 |
| Basic Yellow 23 | 55,48 | -4,84 | 45,42 | 45,68 |
| Basic Yellow 28 | 50,24 | 13,62 | 41,00 | 43,20 |

Ces résultats colorimétriques montrent, que le très court temps de pose sur les cheveux, permet d'obtenir des colorations d'une très bonne intensité.

Exemple 2

Les mèches colorées à l'exemple 1 par le Basic Green 1, le Basic Violet 1, le Basic Violet 2, le Basic Violet 4, le Basic Red 14, le Basic Yellow 13 et le Basic Yellow 23 sont entièrement décapées dans un intervalle de 5 minutes par de l'eau oxygénée à 20 volumes amenée à pH 11 par de la soude.

## Revendications

**1.** Procédé de coloration rapide pour fibres kératiniques humaines, et plus particulièrement les cheveux, consistant à appliquer sur ces fibres au moins une composition tinctoriale contenant dans un milieu approprié pour la teinture au moins un colorant direct choisi parmi les colorants suivants:

- aryle méthaniques
- azoïques cationiques
- méthiniques et azométhiniques
- aziniques,

à maintenir la composition au contact des fibres pendant un temps de pose inférieur à 5 minutes ; puis à rincer les fibres traitées, la coloration présentant selon la notation CIELAB une valeur de L* inférieure à 40 et/ou une valeur de C* supérieure à 20, lorsque la composition est appliquée sur les cheveux à 90% blancs naturels à la température de 27°C ± 5°C pendant une durée de 4 minutes pour un rapport de bain de 10.

**2.** Procédé de coloration rapide pour fibres kératiniques humaines, et plus particulièrement les cheveux, selon la revendication 1, **caractérisé par le fait que** la coloration présente selon la notation CIELAB une valeur de L* inférieure à 40 et/ou une valeur de C* supérieure à 25, lorsque la composition est appliquée sur les cheveux à 90% blancs naturels à la température de 27°C ± 5°C pendant une durée de 4 minutes pour un rapport de bain de 10.

**3.** Procédé de coloration rapide pour fibres kératiniques humaines, et plus particulièrement les cheveux, selon la revendication 1 ou 2, **caractérisé par le fait que** les colorants directs ont pour structure :

- les composés de triaminotriphénylméthane de formule (I) :

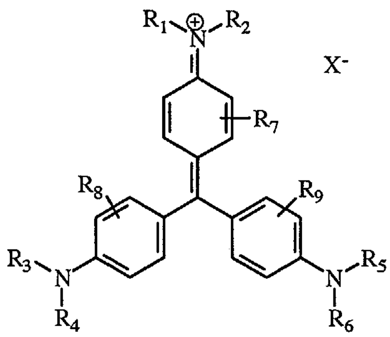

(I)

dans laquelle $R_1$, $R_2$, $R_3$, $R_4$, $R_5$ et $R_6$ indépendamment les uns des autres peuvent être un atome d'hydrogène, un atome d'halogène tel que le chlore, un groupement alkyle en $C_1$-$C_4$ tel que les groupements méthyle ou éthyle ou un groupement mono ou poly hydroxyalkyle en $C_1$-$C_4$ tel que le groupement β-hydroxyéthyle, un groupement phényle ou un groupement benzyle,

$R_7$, $R_8$, $R_9$, indépendamment les uns des autres, peuvent être un atome d'hydrogène, un atome d'halogène tel que le chlore ou un groupement alkyle en $C_1$-$C_4$ tel que les groupements méthyle ou éthyle,

- les composés de diaminotriphénylméthane de formule (II)

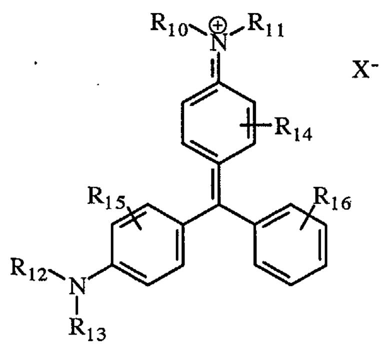

(II)

dans laquelle $R_{10}$, $R_{11}$, $R_{12}$, et $R_{13}$ indépendamment les uns des autres, peuvent être un atome d'hydrogène, un groupement alkyle en $C_1$-$C_4$ tel que les groupements méthyle ou éthyle ou un groupement mono ou poly hydroxyalkyle en $C_1$-$C_4$ tel que le groupement β-hydroxyéthyle,

$R_{14}$ et $R_{15}$, indépendamment l'un de l'autre, peuvent être un atome d'hydrogène ou un groupement alkyle en $C_1$-$C_4$ tel que les groupements méthyle ou éthyle ou un groupement mono ou poly hydroxyalkyle en $C_1$-$C_4$ tel que le groupement β-hydroxyéthyle,

$R_{16}$ peut être un atome d'hydrogène, un atome d'halogène tel que le chlore, un groupement alkyle en $C_1$-$C_4$ tel que les groupements méthyle ou éthyle, ou un groupement mono ou polyhydroxyalkyle en $C_1$-$C_4$ tel que le β-hydroxyéthyle,

- les composés de triaminonaphtyldiphénylméthane de formule (III)

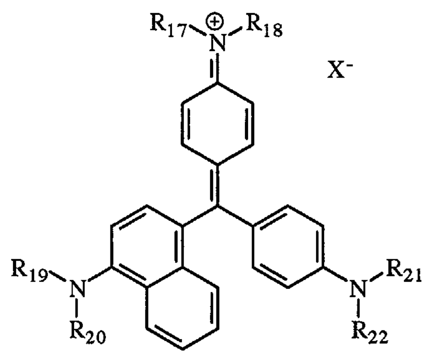

(III)

dans laquelle $R_{17}$, $R_{18}$, $R_{21}$, et $R_{22}$ indépendamment les uns des autres, peuvent être un groupement alkyle en $C_1$-$C_4$ tel que les groupements méthyle, éthyle ou un groupement mono ou poly hydroxyalkyle en $C_1$-$C_4$ tel que le groupement β-hydroxyéthyle,

$R_{19}$ et $R_{20}$, indépendamment l'un de l'autre, peuvent être un atome d'hydrogène, un groupement alkyle en $C_1$-$C_4$ tel que le groupement éthyle, un groupement mono ou poly hydroxyalkyle en $C_1$-$C_4$ tel que le groupement β-hydroxyéthyle, un groupement phényle, un groupement benzyle ou un groupement toluyle,

- des composés de monoaminotriphénylméthane de formule (IV)

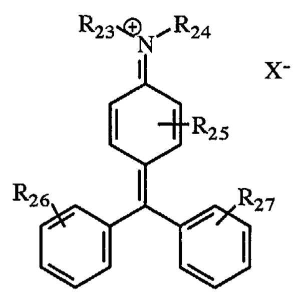

(IV)

dans laquelle $R_{23}$, $R_{24}$, indépendamment l'un de l'autre, peuvent être un atome d'hydrogène, un groupement alkyle en $C_1$-$C_4$ tel que les groupements méthyle ou éthyle, un groupement mono ou poly hydroxyalkyle en $C_1$-$C_4$, ou un groupement benzyle,

$R_{25}$, $R_{26}$ et $R_{27}$, indépendamment les uns des autres, peuvent être un atome d'hydrogène, un atome d'halogène tel que le chlore ou un groupement alkyle en $C_1$-$C_4$ tel que les groupements méthyle ou éthyle,

- les structures des colorants directs azoïques monocationiques et polycationiques de formule (V):

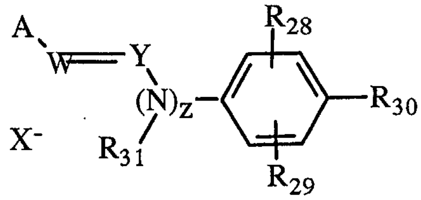

(V)

où A représente l'une des structures suivantes :

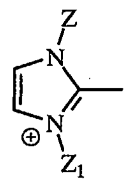 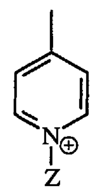 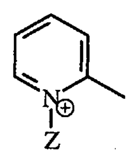 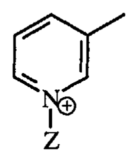 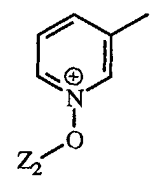

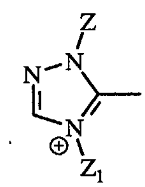 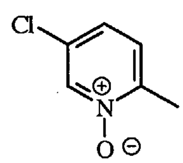

et dans laquelle W et Y représentent indépendamment l'un de l'autre, un atome d'azote ou un atome de carbone,

Z, $Z_1$, $Z_2$ désignent indépendamment les uns des autres un radical alkyle en $C_1$-$C_4$, et en particulier le méthyle,

$R_{28}$ et $R_{29}$, indépendamment l'un de l'autre, représentent un atome d'hydrogène, un atome d'halogène, un groupement alkyle linéaire ou ramifié en $C_1$-$C_4$ ou un groupement alkoxy linéaire ou ramifié en $C_1$- $C_4$, ou un groupement phénol permettant de former un enchaînement naphtalène avec le groupe phényle adjacent,

z est un nombre entier qui peut être 0 ou 1,

$R_{30}$ représente un atome d'hydrogène, un groupement alkoxy linaire ou ramifié en $C_1$-$C_4$, un groupement $NR_{32}R_{33}$ dans lequel $R_{32}$ et $R_{33}$, indépendamment l'un de l'autre, représentent un atome d'hydrogène ou un groupement alkyle linéaire ou ramifié en $C_1$-$C_4$, un groupement toluyle, un groupement mono ou poly hydroxyalkyle tel que le groupement hydroxyéthyle, un groupement $-CH_2SO_3Na$, un groupement benzyle ou forme avec l'atome d'azote adjacent et un atome de carbone du noyau benzénique un hétérocycle,

$R_{31}$ représente un atome d'hydrogène, un groupement alkyle linéaire ou ramifié en $C_1$-$C_4$, ou forme avec l'atome d'azote adjacent et un atome de carbone du noyau benzènique un hétérocycle,

- les structures des colorants méthiniques et azométhiniques de formule (VI):

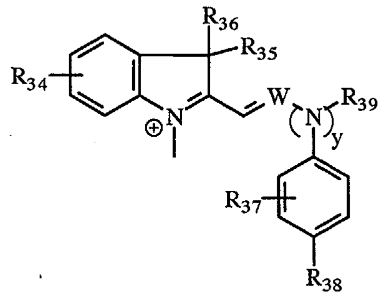

(VI)

dans laquelle $R_{34}$, $R_{35}$ et $R_{36}$, indépendamment les uns des autres, représentent un atome d'hydrogène ou un groupement en $C_1$-$C_4$ tel que le groupement méthyle,

W peut être un atome de carbone ou d'azote,

y représente un nombre entier qui peut être 0 ou 1,

$R_{37}$ peut être un atome d'hydrogène, un groupement alkyle en $C_1$-$C_4$ tel que le groupement méthyle, ou un groupement alkoxy en $C_1$-$C_4$ tel que le groupement méthoxy,

$R_{38}$ peut être un atome d'hydrogène, un groupement méthoxy, un groupement $NR_{40}R_{41}$ où $R_{40}$ et $R_{41}$, indépendamment l'un de l'autre, peuvent être un groupement alkyle en $C_1$-$C_4$ tel que méthyle, éthyle ou propyle éventuellement substitué par un atome de chlore ou par un groupement cyano,

$R_{39}$ peut être un atome d'hydrogène, un groupement alkyle en $C_1$-$C_4$ tel que le groupement méthyle, ou forme un hétérocycle avec les atomes de carbone du noyau benzénique adjacent,

- le colorant méthinique Basic Orange 21 de formule (VII)

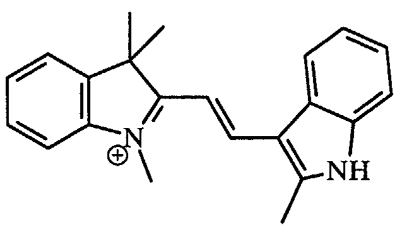

(VII)

- la carbocyanine de formule (VIII) :

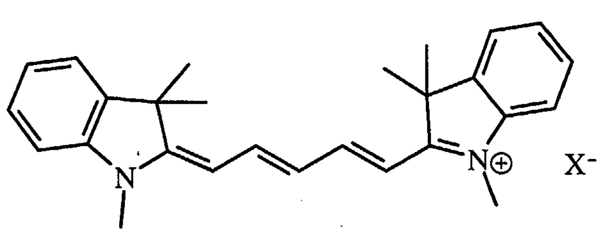

(VIII)

- les dérivés de benzothiazole de formule (IX) :

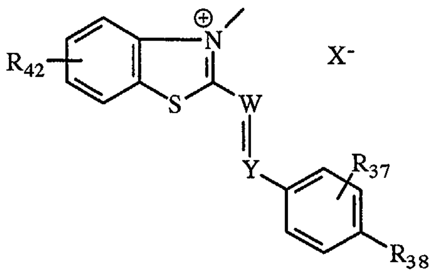

(IX)

dans laquelle $R_{42}$ peut être un atome d'hydrogène, un groupement alkyle en $C_1$-$C_4$ tel que méthyle ou éthyle, un groupement alcoxy en $C_1$-$C_4$ tel que le groupement méthoxy,

$R_{37}$ et $R_{38}$ ont la même signification que dans la structure de formule (VI),

W et Y, indépendamment l'un de l'autre, peut être un atome de carbone ou d'azote,

- les phénazines de formule (X) :

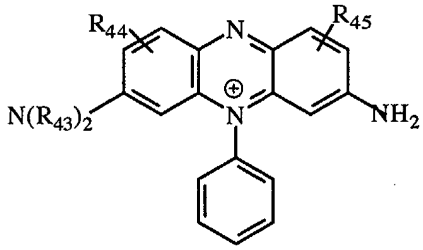

(X)

$R_{43}$, $R_{44}$ et $R_{45}$, indépendamment les uns des autres, représentent un atome d'hydrogène, un groupement alkyle en $C_1$-$C_4$ tel que méthyle ou éthyle,

- les azophénazines de formule (XI) :

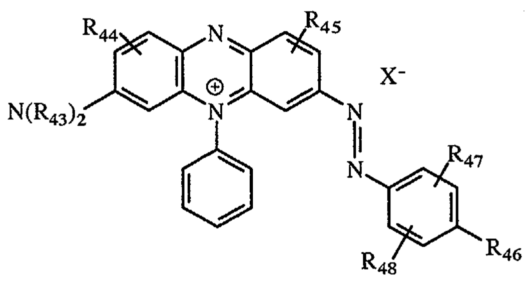

(XI)

dans laquelle $R_{43}$, $R_{44}$ et $R_{45}$ ont la même signification que dans la formule (X),

$R_{46}$ représente un atome d'hydrogène, un groupement hydroxyle ou un groupement amino,

$R_{47}$ et $R_{48}$ peuvent être un atome d'hydrogène, un groupement hydroxyle ou un groupement phényle permettant de former un enchaînement naphtalène avec le groupement phényle adjacent,

- les thiazines de formule (XII) :

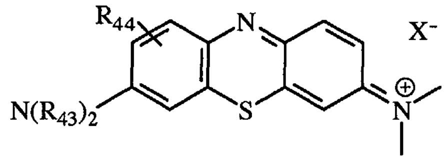

(XII)

dans laquelle $R_{43}$ et $R_{44}$ ont la même signification que dans la formule (X),

- les oxazines de formule (XIII) :

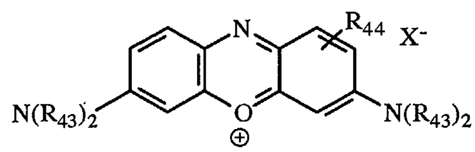

(XIII)

dans laquelle $R_{43}$ et $R_{44}$ ont la même signification que dans la formule (X),
dans chacune des structures ci-dessus, $X^-$ représente un anion qui peut être issu d'un atome d'halogène, préférentiellement l'atome chlore, ou les ions $HSO_4^-$, méthosulfate, benzoate ou acétate.

4. Procédé de coloration rapide pour fibres kératiniques humaines, et plus particulièrement les cheveux, selon la revendication 1, **caractérisé par le fait que** le temps de pose est compris entre 1 et 3 minutes.

5. Procédé de coloration rapide pour fibres kératiniques humaines, et plus particulièrement les cheveux, selon la revendication 4, **caractérisé par le fait que** le temps de pose est compris entre 1 et 2 minutes.

6. Procédé de coloration rapide pour fibres kératiniques humaines, et plus particulièrement les cheveux, selon l'une quelconque des revendications 1 à 5, **caractérisé par le fait que** la concentration de la composition en colorants directs est comprise entre 0,001 et 10%, et préférentiellement entre 0,05 et 5%.

7. Procédé de coloration rapide pour fibres kératiniques humaines, et plus particulièrement les cheveux, selon l'une quelconque des revendications 1 à 6, **caractérisé par le fait que** la gamme de pH pour la formulation est comprise entre 2 et 11, préférentiellement entre 3 et 11.

8. Procédé de coloration rapide pour fibres kératiniques humaines, et plus particulièrement les cheveux, selon l'une quelconque des revendications 1 à 7, **caractérisé par le fait que** la température d'application est comprise entre la température ambiante et 80°C, et préférentiellement entre la température ambiante et 60°C, et plus particulièrement les cheveux sont teints à une température de 27°C ± 5°C.

9. Procédé de décapage rapide pour fibres kératiniques humaines, et plus particulièrement les cheveux, **caractérisé par le fait que** le décapage de la fibre colorée par le procédé décrit dans l'une quelconque des revendications précédentes est réalisé avec un temps de pose inférieur à 5 minutes par un ou plusieurs composés choisis parmi les agents oxydants ou réducteurs.

10. Procédé de décapage rapide pour fibres kératiniques humaines, et plus particulièrement les cheveux selon la revendication 9, **caractérisé par le fait que** lesdites fibres à décaper ont été préalablement colorées par les colorants directs choisis parmi le Basic Blue 1, le Basic Blue 5, le Basic Green 1, le Basic Green 4, le Basic Red 9, le Basic Violet 1, le Basic Violet 2, le Basic Violet 3, le Basic Violet 4, le Hofmann's Violet, le Opal Blue SS, le Basic Orange 21, le Basic Red 13, le Basic Red 14, le Basic Violet 16 et le Basic Violet 7.

11. Procédé de décapage rapide pour fibres kératiniques humaines, et plus particulièrement les cheveux selon la revendication 9 ou 10, **caractérisé par le fait que** ce procédé utilise au moins un agent oxydant et s'effectue à pH alcalin.

12. Procédé de décapage rapide pour fibres kératiniques humaines, et plus particulièrement les cheveux selon la revendication 9 ou 10, **caractérisé par le fait que** ce procédé utilise au moins un agent réducteur et s'effectue à pH acide.

Office européen des brevets

# RAPPORT PARTIEL DE RECHERCHE EUROPEENNE

qui selon la règle 45 de la Convention sur le brevet européen est consideré, aux fins de la procédure ultérieure, comme le rapport de la recherche européenne

Numéro de la demande
EP 03 29 2596

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 818 536 A (OREAL) 28 juin 2002 (2002-06-28) * page 10, ligne 1 - page 13, ligne 17; revendication 59 * --- | 1-12 | A61K7/13 |
| X | EP 0 796 850 A (OREAL) 24 septembre 1997 (1997-09-24) * page 12, ligne 24 - ligne 49 * * page 13, ligne 53 - ligne 54; revendication 17 * --- | 1-12 | |
| E | WO 02 100367 A (OREAL ;PLOS GREGORY (FR); SAMAIN HENRI (FR)) 19 décembre 2002 (2002-12-19) * page 13, ligne 9 - page 17, ligne 12; revendications 16,18 * --- | 1-12 | |
| A | FR 2 070 879 A (CIBA GEIGY AG) 17 septembre 1971 (1971-09-17) * page 1, ligne 31 - ligne 32 * * page 7, ligne 26 - ligne 28 * --- | 1-12 | |
| | -/-- | | |

DOMAINES TECHNIQUES RECHERCHES (Int.Cl.7)

A61K

## RECHERCHE INCOMPLETE

*La division de la recherche estime que la présente demande de brevet, ou une ou plusieurs revendications,* ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| BERLIN | 23 janvier 2004 | Boeker, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

Office européen des brevets

**RECHERCHE INCOMPLETE FEUILLE SUPPLEMENTAIRE C**

Numéro de la demande
EP 03 29 2596

Revendications ayant fait
l'objet de recherches complètes:
-

Revendications ayant fait
l'objet de recherches incomplètes:
1-12

Raison pour la limitation de la recherche:

Les revendications 1-12 présentes ont trait à une méthode défini en faisant référence à une caractéristique ou propriété souhaitable, à savoir: une coloration présentant selon la notation CIELAB une valeur de L* inférieure à 40 et/ou une valeur de C* supérieure à 20, lorsque la composition est appliquée sur les cheveux à 90% blancs naturels à la température de 27 C +/- 5 C pendant une durée de 4 minutes pour un rapport de bain de 10.

Les revendications couvrent tous les méthodes aboutissant à des produits présentant cette caractéristique ou propriété, alors que la demande ne fournit un fondement au sens de l'Article 84 CBE et/ou un exposé au sens de l'Article 83 CBE que pour un nombre très limité de tels méthodes. Dans le cas présent, les revendications manquent de fondement et la demande manque d'exposé à un point tel qu'une recherche significative sur tout le spectre couvert par les revendications est impossible.

Indépendamment des raisons évoquées ci-dessus, les revendications manquent aussi de clarté. En effet, on a cherché à définir la méthode au moyen du résultat à atteindre. Ce manque de clarté est, dans le cas présent, de nouveau tel qu'une recherche significative sur tout le spectre couvert par les revendications est impossible. En conséquence, la recherche n'a été effectuée que pour les parties des revendications dont l'objet apparaît être clair, fondé et suffisamment exposé, à savoir les parties concernant les colorants directs mentionnés dans les revendications 1 et 3.

Office européen des brevets

# RAPPORT PARTIEL DE RECHERCHE EUROPEENNE

Numero de la demande
EP 03 29 2596

| DOCUMENTS CONSIDERES COMME PERTINENTS | | | CLASSEMENT DE LA DEMANDE (Int.Cl.7) |
|---|---|---|---|
| Catégorie | Citation du document avec indication, en cas de besoin, des parties pertinentes | Revendication concernée | |
| A | WO 95 15144 A (CIBA GEIGY AG ;MOECKLI PETER (CH)) 8 juin 1995 (1995-06-08)<br>* exemples *<br>----- | 1-12 | |
| | | | DOMAINES TECHNIQUES RECHERCHES (Int.Cl.7) |

EPO FORM 1503 03.82 (P04C11)

**ANNEXE AU RAPPORT DE RECHERCHE EUROPEENNE RELATIF A LA DEMANDE DE BREVET EUROPEEN NO.** EP 03 29 2596

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-01-2004

| Document brevet cité au rapport de recherche | | Date de publication | Membre(s) de la famille de brevet(s) | | | Date de publication |
|---|---|---|---|---|---|---|
| FR 2818536 | A | 28-06-2002 | FR | 2818536 | A1 | 28-06-2002 |
| | | | EP | 1345576 | A1 | 24-09-2003 |
| | | | FR | 2818544 | A1 | 28-06-2002 |
| | | | WO | 02051366 | A1 | 04-07-2002 |
| EP 0796850 | A | 24-09-1997 | FR | 2746392 | A1 | 26-09-1997 |
| | | | DE | 69700350 | D1 | 02-09-1999 |
| | | | DE | 69700350 | T2 | 18-11-1999 |
| | | | EP | 0796850 | A1 | 24-09-1997 |
| | | | US | 5931973 | A | 03-08-1999 |
| WO 02100367 | A | 19-12-2002 | FR | 2825624 | A1 | 13-12-2002 |
| | | | WO | 02100367 | A1 | 19-12-2002 |
| FR 2070879 | A | 17-09-1971 | AT | 310949 | B | 25-10-1973 |
| | | | BE | 760346 | A1 | 15-06-1971 |
| | | | CA | 951244 | A1 | 16-07-1974 |
| | | | CH | 545626 | A | 15-02-1974 |
| | | | DE | 2058940 | A1 | 24-06-1971 |
| | | | ES | 386463 | A1 | 01-08-1974 |
| | | | FR | 2070879 | A1 | 17-09-1971 |
| | | | NL | 7018286 | A | 18-06-1971 |
| | | | ZA | 7008309 | A | 29-09-1971 |
| WO 9515144 | A | 08-06-1995 | AU | 671394 | B2 | 22-08-1996 |
| | | | AU | 8144794 | A | 19-06-1995 |
| | | | BR | 9405984 | A | 06-02-1996 |
| | | | CA | 2153332 | A1 | 08-06-1995 |
| | | | CN | 1117265 | A ,B | 21-02-1996 |
| | | | DE | 69422799 | D1 | 02-03-2000 |
| | | | DE | 69422799 | T2 | 31-08-2000 |
| | | | WO | 9515144 | A1 | 08-06-1995 |
| | | | EP | 0681464 | A1 | 15-11-1995 |
| | | | ES | 2143033 | T3 | 01-05-2000 |
| | | | JP | 8507545 | T | 13-08-1996 |
| | | | US | 5888252 | A | 30-03-1999 |
| | | | ZA | 9409469 | A | 30-05-1995 |

EPO FORM P0460

Pour tout renseignement concernant cette annexe : voir Journal Officiel de l'Office européen des brevets, No.12/82